# FASCICULE DE BREVET EUROPEEN

(11) **EP 0 550 724 B1**
(45) Date de publication et mention de la délivrance du brevet: **06.03.1996**
(21) Numéro de dépôt: 92915893.9
(22) Date de dépôt: 22.07.1992
(51) Int. Cl.: A61K 39/118

(54) **Composition vaccinale pour prévenir chez l'animal l'avortement causé par Chlamydia psitacci**
Impfstoff-Zubereitung zur Vorbeugung gegen bei Tieren durch Chlamydia psitacci verursachte Fehlgeburt
Vaccine composition for preventing animal abortion caused by Chlamydia psitacci

(30) Priorité: 25.07.1991 CH 2227/91
(43) Date de publication de la demande: 14.07.1993
(73) Titulaire: LIOTET, Serge, F-75011 Paris (FR); CATALAN, François, F-75018 Paris (FR); GOUCHET, Frank, F-45450 Donnery (FR)
(72) Inventeur: LIOTET, Serge, F-75011 Paris (FR); CATALAN, François, F-75018 Paris (FR); GOUCHET, Frank, F-45450 Donnery (FR)
(74) Mandataire: Micheli & Cie
(86) Numéro de dépôt international: EP9201637
(87) Numéro de publication internationale: WO9301832

(56) Documents cités:
- WO-A-92/00096
- GB-A- 2 057 881
- GB-A- 2 092 443
- EMBASE ABREGE NO 82041115, A.P. JOHNSON ET AL.:"IMMUNITY TO REINFECTION OF THE GENITAL TRACT OF MARMOSETS WITH CHLAMYDIA TRACHOMATIS".
- BIOLOGICAL ABSTRACTS vol. 62, no. 10 , 1976, Philadelphia, PA, US; abstract no.
- 54909, SAYED, H. ET AL. 'DIFFERENCES IN PHYSICOCHEMICAL AND ANTIGENIC PROPERTIES OF CHLAMYDIAL STRAINS.' page 5412 ;

## Description

L'invention a pour objet une composition vaccinale, plus particulièrement une composition destinée à la prophylaxie, par stimulation antigénique orale, des avortements chlamydiosiques chez les animaux d'élevage.

On sait depuis plusieurs années déjà que les avortements épizotiques chez les animaux d'élevage tels les ovins sont consécutifs à une infection bactérienne, plus particulièrement des bactéries de l'espèce Chlamydia psittaci.

Sur de telles bases, divers auteurs ont proposé la préparation de vaccins appropriés à base de bactéries du type Chlamydia psittaci tuées ou tout au moins convenablement atténuées. On a proposé par exemple (FR-A-2.507.479) des vaccins se composant de suspensions inactivées de sac vittelins d'embryons de poulets infectés, de l'espèce pathogène, Chlamydia pstttaci, avec addition d'un adjuvant adéquat. D'autres (GB-A-2.092.443), ont proposé des vaccins à base de suspensions aqueuses, inactivées au formaldéhyde, de corps élémentaires de l'espèce Chlamydia psittaci cultivés sur fibroblastes. Dans un cas comme dans l'autre, de telles compositions sont destinées à une administration par voie parentérale, intramusculaire ou sous-cutanée de préférence.

Chlamydia psittaci étant fortement pathogène pour les espèces animales considérées, l'emploi de bactéries atténuées ou encore mieux tuées est indispensable à la réalisation de vaccins appropriés. Cependant, l'inconvénient de vaccins à base de bactéries atténuées réside dans leur faible pouvoir immunogène, l'immunité insuffisante qu'ils confèrent aux animaux à protéger et, parfois, la difficulté d'administration de la composition étant donné la nature huileuse des adjuvants utilisés. De plus, la propagation des bactéries pathogènes sur sacs vitellins embryonnaires ou sur membranes foetales de mammifères est une technique onéreuse et difficile à standardiser.

Pour ce qui est des vaccins à base de Chlamydia psittaci tuées, la littérature ne donne aucune information précise, mais les connaissances actuelles laissent à penser que dans de tels cas il y a perte du pouvoir immunogène aussi bien que du pouvoir pathogène. On constate ainsi que l'homme du métier est à ce jour dépourvu de moyens prophylactiques efficaces et sûrs d'emploi pour lutter contre les avortements chlamydiosiques des animaux d'élevage.

L'invention a pour but de permettre de surmonter avantageusement les inconvénients des vaccins connus à ce jour dans ce domaine. La dite invention est notamment basée sur le fait que, contrairement à toute attente, l'immunisation au moyen de germes de l'espèce Chlamydia trachomatis protège efficacement contre une infection à pstttaci : cette immunisation croisée n'a jamais été observée pour de telles bactéries, en particulier sur les espèces animales retenues. L'invention a en outre pour mérite l'administration aux animaux d'élevage de bactéries non pathogènes pour ceux-ci, à savoir C. trachomatis qui, dans les conditions d'administration, présentent cependant un pouvoir immunogène suffisant, aussi bien tuées que vivantes. Enfin, la dite invention propose une composition vaccinale permettant la stimulation antigénique orale, une voie particulièrement intéressante encore non explorée chez les espèces animales considérées.

L'objet de l'invention consiste donc plus précisément en une composition vaccinale destinée à la prophylaxie par stimulation antigénique orale des avortements à Chlamydia psittaci chez les animaux d'élevage, qui est caracterisée en ce qu'elle contient des bactéries de l'espèce Chlamydia trachomatis tuées ou vivantes présentant au moins l'un des sérotypes A à L de ladite espèce

Les avortements à Chlamydia psittaci se produisent, plus particulièrement chez les ovins, où cette affection entraîne des pertes considérables dans le cheptel. De façon surprenante, on a observé de bons résultats en partant de germes pathogènes humains de sérotype A à C (C. trachomatis, responsable du trachome), ou D à K (C. trachomatis, responsable d'affections oculo- ou urogénitales humaines) ou encore L (C. trachomatis, responsable de la lymphogranulomatose), utilisant aussi bien un sérotype isolé, le sérotype D par exemple, qu'un mélange de plusieurs sérotypes. Il revient à l'homme du métier de sélectionner le ou les sérotypes les plus appropriés.

Etant donné leur absence de pouvoir pathogène pour les espèces animales considérées, les germes C. trachomatis peuvent entrer dans la composition vaccinale sous forme vivante ou tuée, toutes deux conservant un pouvoir immunogène suffisant. S'agissant de germes C. trachomatis tués, d'emploi nettement plus aisé, il a été observé, de façon surprenante, que les germes lyophilisés étaient en fait des germes tués ayant conservé tout leur pouvoir immunogène.

Selon l'invention, la composition vaccinale peut avantageusement contenir un diluant ou excipient inerte pharmaceutiquement acceptable et, le cas échéant, un adjuvant ou stimulant immunologique adéquat, ou encore un adjuvant stimulant l'activité des macrophages. Une telle énumération n'est cependant pas exhaustive.

La composition selon l'invention s'administre par voie orale; elle peut se présenter sous toute forme galénique appropriée, par exemple sous la forme de suspension de bactéries vivantes ou de leur lyophilisat, sous une forme galénique gastro-résistante telle une gélule ou un comprimé gastro-résistant, sous forme de pastille ou de comprimé autocollant à faire fondre dans la bouche ou encore sous forme de microsphères, nanosphères, microcapsules ou nanocapsules biodégradables, ou encore sous forme de vésicules lipidiques ou de liposomes. Il est du ressort de l'homme du métier de déterminer la forme galénique la plus aédquate, en fonction de l'espèce animale concernée, en particulier de son tractus digestif.

Pour obtenir l'immunisation souhaitée, il convient d'administrer la composition de manière répétée, sur une période de 15 à 30 jours et d'effectuer un rappel après 3 mois, le cas échéant. Par exemple, on peut administrer la dose prescrite à J₁, J₂, J₃ puis à J₁₅, J₁₆ et J₁₇ et effectuer ensuite un rappel à 90 jours, ou encore à J₁, J₁₅ et J₃₀, les rappels subséquents étant également effectués à J₉₀. Bien entendu, tout autre rythme d'administration peut être envisagé, tenant compte notamment des période de rut, de gestation ou de mise bas des espèces animales concernées.

L'efficacité de compositions conformes à l'invention peut être illustrée comme suit. Cette illustration n'est en aucun cas limitative.

### a. Mise en évidence du pouvoir immunogène de germes Chlamydia trachomatis lyophilisée (lapin)

**a.1** On a préparé une suspension antigénique à partir de culture de C. trachomatis sérotype D en procédant comme suit : les antigènes sont obtenus en culture de Chlamydia sur cellules McCOY ou HELA 229, et comportent un mélange de corps réticulés et de corps élémentaires. Ces cellules sont remises en suspension et ultrasoniquées ou séparées par tout autre système mécanique.
Les anticorps on été recherchés et dosés dans le sang des lapins à partir du 8ème jour après administration orale de la suspension antigénique, l'absence d'anticorps ayant été vérifiée avant toute injection. Les anticorps se positivent dès le 8ème jour à un titre faible dans le sérum (1/4 à 1/8) et se précisent franchement dès le 15ème jour (3ème détermination : J₁, J₈, J₁₅).
Le tableau suivant donne les titres observés pour cinq lapins répartis dans des cages 114, 115, 116, 117, et 119, en fonction du temps écoulé depuis l'immunisation

| **LAPIN** | 114 | 115 | 116 | 117 | 119 |
|---|---|---|---|---|---|
| avant immunisation | 0 | 0 | 0 | 0 | 0 |
| J12 | 1/16 | 1/256 | 1/128 | 1/16 | 1/4 |
| J30 | 1/2048 | 1/32768 | 1/16384 | 1/8192 | 1/1024 |
| J120 | 1/4096 | 1/8192 | 1/1024 | * | * |
| * : Titrage non effectué | | | | | |

On a ainsi pu observer que l'ingestion sur trois jours consécutivement, à deux reprises, de particules chlamydiennes vivantes entraîne chez le lapin l'apparition d'anticorps spécifiques à un titre élevé dans le sérum sanguin prélevé.
**a.2** On a préparé séparément deux suspensions antigéniques à partir d'une culture de C. trachomatis sérotype D conformément au processus décrit sous a.1.
La première suspension, contenant les germes vivants, a été utilisée telle que pour l'expérimentation La seconde suspension a été soumise à lyophilisation, ce qui a conduit à l'obtention de germes tués comme l'ont confirmé des essais de culture subséquents.
La mise en évidence de l'apparition d'anticorps a été effectuée en parallèle sur deux groupes de 5 lapins chacun, l'un recevant les germes vivants, l'autre les germes lyophilisés. L'administration des antigènes Chlamydia a été effectuée par tubage gastrique comme précédemment et les prélèvements de sérum ont été pratiqués à J₀, J₇, J₁₄, J₂₁ et J₂₈.
Selon la technique du Western-Blot, les échantillons de sérum prélevés comme ci-dessus, montrent l'apparition d'anticorps anti-Chlamydia à partir de J₁₄, ceux-ci atteignant un maximum de variété a J₂₁. L'expérimentation permet en outre de préciser que les résultats sont identiques, qu'ils soient obtenus a partir de germes vivants ou lyophilisé, tant du point de vue quantitatif que qualitatif.

### b. Mise en évidence du pouvoir immunogène de Chlamydia trachomatis chez la brebis

### b.1 Recherche de l'effet-dose

Pour cette expérimentation préalable, on a recruté 30 brebis d'environ 10 mois, de race rustique, exemptes d'anticorps anti-Chlamydia (tolérance jusqu'à un taux de dilution de 1/32).

Sur chacune des brebis, on a ensuite procédé à la pose d'une canule duodénale, en procédant selon les techniques usuelles en expérimentation vétérinaire.

La composition vaccinale de base consiste en une suspension de germes Chlamydia trachomatis sérotype D lyophilisés, de concentration initiale 10⁸/ml. Par dilutions successives, on a préparé diverses doses de références, allant de 10⁸ à 10³/ml, chacune de ces dilutions étant administrée à un groupe de 3 brebis.

La vaccination proprement dite s'effectue par injection de la suspension directement dans la canule duodénale, de sorte que la totalité de la suspension passe dans la circulation duodénale. La vaccination a été effectuée à J₁, J₂, J₃ suivis de J₁₅, J₁₆ et J₁₇, J₁ étant le premier jour de la manipulation.

Des prélévements ont été effectués sur les brebis testées comme sur les brebis témoins, respectivement à J₁, J₇, J₁₄, J₂₁ et J₂₈. Les prélévements furent de 2 ordres : sérum sanguin et mucus vaginal.

Sur la base des résultats observés, on a déterminé la dose vaccinante optimale, pour la voie d'administration retenue, à env. 10⁶ germes / ml. Elle sera utilisée pour les expérimentations décrites ci-après.

### c. Mise en évidence d'une protection immunologique anti-Chlamydia psittaci chez la brebis

### c.1 Préparation et sélection des brebis

7 brebis gestantes, de race rustique, ont été retenues pour expérimentation. 5 d'entre elles ont été vaccinées à l'aide de Chlamydia trachomatis lyophilisés par voie duodénale, selon la méthode décrite sous b.1.
Les dites brebis se répartissent comme suit :

| Brebis no | C. tracomatis Germes/ml | Anti-corps sérum |
|---|---|---|
| 64R | 104 | 1/16 |
| 65R | 105 | 1/64 |
| 84R | 103 | 1/32 |
| 86R | 108 | 1/256 |
| 97R | 104 | 1/256 |
| 29V | - | - 1) |
| 88R | - | - 2) |

| | | |
|---|---|---|
| 1) témoin non vacciné, à sérologie négative, considéré comme témoin positif 2) témoin non vacciné, à sérologie négative considéré | | |
| comme témoin négatif. | | |

En définissant par J₀ le jour de l'épreuve par inoculation de Chlamydia psittaci vivant (challenge), le schéma de vaccination des brebis a été réalisé comme suit :
J-165, J-150, J-90 ,
les taux d'anti-corps sériques figurant dans le tableau ci-dessus étant ceux mesurés à J-1..

Toutes les brebis retenues pour l'expérimentation se situent dans le dernier tiers de leur gestation, soit à 100 jours ou plus de gestation.

### c.2 Mise à l'épreuve

La mise à l'épreuve des brebis référencées sous c.1 a été effectuée au moyen d'une suspension aqueuse de Chlamydia psittaci préparée selon les techniques usuelles, à partir de souches cultivées sur membranes vitellines. La dite suspension contient de 10⁵ à 10⁷ corps infectants / ml.

A l'exception du sujet "88R" (témoin négatif) chaque brebis du groupe a reçu, par voie intradermique pratiquée au tiers moyen de l'encolure, 1 ml de suspension telle que définie plus haut.

### c.3 Examen clinique

Chacune des brebis a subi un examen clinique journalier et un examen échographique avant le challenge (J₀) et 8 jours après le dit challenge.

L'examen échographique porte en particulier sur la croissance foetale, déterminée par la mesure du diamètre thoracique abdominal (DTA), le rythme cardiaque du foetus considéré comme normal à 140-180 battements/min, ainsi que ses mouvements.

Les diverses observations effectuées sont rassemblées sous forme de tableaux ci-après.

| **Variation de la température (°C) rectale au cours du challenge** | | | | | | |
|---|---|---|---|---|---|---|
| **No** | **J-1** | **J0** | **J1** | **J2** | **J4** | **J5** |
| 64R | 38.6 | 39.1 | 41.8 | 41.3 | 38.7 | 39 |
| 65R | 38.8 | 39.6 | 40.8 | 40.9 | 39.2 | 39.5 |
| 84R | 38.7 | 40. | 40.8 | 40.2 | 40.1 | 38.7 |
| 86R | 38.8 | 39.7 | 40.3 | 41.6 | 39.2 | 38.8 |
| 97R | 38.9 | 39.7 | 39.8 | 41.1 | 39.3 | 38.9 |
| 29V | 38.7 | 38.5 | 41.0 | 41.2 | 39.4 | 39.6 |
| 88R | 38.9 | - | - | 39.4 | 39.4 | 39.7 |
| Note : La température rectale à J₀ a été prise 12 heures après le challenge. | | | | | | |

On observe un pic thermique entre 24 et 48 heures après la mise à l'épreuve avec une différence de près de 2°C par rapport à une situation normale; la température redescend ensuite à une valeur subnormale à J₅.

| **Contrôle de gestation à J-1** Résultats Echographiques | | | | |
|---|---|---|---|---|
| No | Mouvements | Rythme cardiaque | DTA en mm | Nb foetus |
| 64R | * | N | 70 | 1 |
| 65R | * | N | 73 | 1 |
| 84R | * | N | 71 | 1 |
| 86R | * | N | non mesuré | 1 |
| 97R | * | N | 54 | 1 |
| 29V | * | N | 66 | 1 |
| 88R | * | N | 40 | 1 |
| N = normal (140-180/min) * = normaux | | | | |

| **Bilan et contrôle de gestation à J₆** Résultats Echographiques | | | |
|---|---|---|---|
| No | Mouvements | Rythme Cardiaque | Naissance ou avortement |
| 64R | * | N | |
| 65R | - | - | Avortement à J₄ |
| 84R | * | N | |
| 86R | * | N | |
| 97R | * | N | |
| 29V | - | - | Avortement à J₂ |
| 88R | * | N | |

| **Bilan et contrôle de gestation à J₃₀** Résultats Echographiques | | | |
|---|---|---|---|
| No | Mouvements | Rythme Cardiaque | Naissance ou avortement |
| 64R | - | - | Agneau normal vivant à J₂₈ |
| 65R | - | - | Avortement à J₄ |
| 84R | - | - | Agneau normal vivant à J₃₀ |
| 86R | * | N | |
| 97R | * | N | |
| 29C | - | - | Avortement à J₂ |
| 88R | * | N | |

| **Bilan final à J₆₀** | |
|---|---|
| No | Naissance ou avortement |
| 64R | Agneau normal vivant à J₂₈ |
| 65R | Avortement à J₄ |
| 84R | Agneau normal vivant à J₃₀ |
| 86R | Agneau normal vivant à J ₄₃ |
| 97R | Agneau normal vivant à J₄₁ |
| 29C | Avortement à J₂ |
| 88R | Agneau normal vivant à J₅₂ |

### Remarques

1.- la réaction à l'injection intradermique d'environ 10⁶ Chlamydiae psittaci s'est traduite par un pic thermique se situant à plus de 41°C en moyenne pour le lot mis à l'épreuve, ce qui est conforme à la littérature et signe d'une réelle infection à psittaci. Dans le même temps la brebis 88R (témoin négatif) conserve une température et un habitus normal.
2.- deux avortements ont eu lieu dans les heures suivant le challenge, le premier à 48 heures sur le témoin positif 29R, le second à J₄; une échographie à J₆ confirme la bonne activité des embryons sur les autres brebis.
3.- deux naissances ont eu lieu à J₂₈ et J₃₀; les agneaux étaient parfaitement normaux et vivants. A J₃₀ les autres foetus sont vivants et la gestation se poursuit normalement.
4.- 3 autres naissances on eu lieu à J₄₁, J₄₃ et J₅₂ respectivement, ces brebis ayant toutes mis bas un agneau normal et très vigoureux.

### Conclusion

La protection conférée par le vaccin à C. trachomatis lyophilisé s'est révélée satisfaisante dans 4 cas sur 5 lorsque les brebis sont soumises à un challenge avec C. psittaci vivant. On peut ainsi considérer que la vaccination confère une bonne protection croisée, notamment lorsque la réponse sérologique des animaux vaccinés atteint ou dépasse le seuil de 1/128. Il est à noter que 2 animaux sur 3 ayant une réponse sérologique faible, inférieure ou égale à 1/64, ont vu néanmoins leur gestation maintenue: on peut donc considérer à priori que la protection est efficace, ce qui signifie, en termes de biologie, que 70% des animaux devraient être protégés au moins.

La vaccination par voie orale de Chlamydia trachomatis lyophilisé confère une immunité satisfaisante alors qu'à ce jour aucun vaccin Chlamydia n'est actif par cette voie.

## Revendications

1. Composition vaccinale destinée à la prophylaxie par stimulation antigénique orale des avortements à Chlamydia psittaci chez les animaux d'élevage, caractérisée en ce qu'elle contient des bactéries de l'espèce Chlamydia trachomatis tuées ou vivantes présentant au moins l'un des sérotypes A à L de ladite espèce.

2. Composition selon la revendication 1, caractérisée en ce que les animaux d'élevage sont les ovins.

3. Composition selon la revendication 1 ou 2, caractérisée en ce qu'elle contient des bactéries appartenant au moins à l'un des sérotypes A à C, ou D à K, soit au sérotype L.

4. Composition selon la revendication 1, caractérisée en ce que les bactéries tuées sont des bactéries lyophilisées.

5. Composition selon la revendication 1, caractérisée en ce que les bactéries vivantes comportent un mélange de corps réticulés et de corps élémentaires.

6. Composition selon l'une des revendications 1 à 5, caractérisée en ce qu'elle contient outre un excipient inerte, un adjuvant ou stimulant immunologique ou encore un adjuvant stimulant l'activité des macrophages.

7. Composition selon l'une des revendications 1 à 6, caractérisée en ce qu'elle se présente sous une forme galénique orale gastro-résistante.

8. Composition selon l'une des revendications 1 à 7, caractérisée en ce qu'elle se présente sous forme de microsphères, nanosphères, microcapsules ou nanocapsules biodégradables, ou encore sous forme de vésicules lipidiques ou de liposomes.

## Claims

1. Vaccine composition intended for preventing Chlamydia psittaci abortions in livestock by oral antigenic stimulation, characterized in that it contains killed or live bacteria of the species Chlamydia trachomatis presenting at least one of the serotypes A to K of said species.

2. Composition according to claim 1, characterized in that livestock is sheep.

3. Composition according to claim 1 or 2, characterized in that it contains bacteria of at least one of the serotypes A to C, or D to K, or of the serotype L.

4. Composition according to claim 1, characterized in that the killed bacteria are lyophilized bacteria.

5. Composition according to claim 1, characterized in that the live bacteria comprise a mixture of cross-linked bodies and elementary bodies.

6. Composition according to one of claims 1 to 5, characterized in that it contains in addition an inert excipient, an immunological adjuvant or stimulant, or also a macrophagic activity-stimulating adjuvant.

7. Composition according to one of claims 1 to 6, characterized in that it is presented in a gastro-resistant oral galenical form.

8. Composition according to one of claims 1 to 7, characterized in that it is presented in the form of biodegradable microspheres, nanospheres, microcapsules or nanocapsules, or also in the form of lipidic vesicles or of liposomes.

## Patentansprüche

1. Impfstoffzusammensetzung, bestimmt zur auf oraler Antigenstimulierung beruhenden Vorbeugung von durch *Chlamydia psittaci* hervorgerufenen Aborten bei Zuchttieren, dadurch gekennzeichnet, dass sie abgetötete oder lebende Bakterien der Gattung *Chlamydia trachomatis* enthält, die zumindest einen der Serotypen A bis L der benannten Gattung darstellen.

2. Zusammensetzung gemäss Anspruch 1, dadurch gekennzeichnet, dass die Zuchttiere Schafe sind.

3. Zusammensetzung gemäss Anspruch 1 oder 2, dadurch gekennzeichnet, dass sie Bakterien enthält, die zumindest einem der Serotypen A bis C oder D bis K oder aber dem Serotyp L angehören.

4. Zusammensetzung gemäss Anspruch 1, dadurch gekennzeichnet, dass die abgetöteten Bakterien lyophilisierte Bakterien sind.

5. Zusammensetzung gemäss Anspruch 1, dadurch gekennzeichnet, dass die lebenden Bakterien aus einer Mischung von vernetzten und elementaren Körpern bestehen.

6. Zusammensetzung gemäss Ansprüchen 1 bis 5, dadurch gekennzeichnet, dass sie ausserdem ein inertes Bindemittel, ein immunologisches Adjuvans oder Stimulans oder aber ein die Aktivität der Makrophagen anregendes Adjuvans enthält.

7. Zusammensetzung gemäss einem der Ansprüche 1 bis 6, dadurch gekennzeichnet, dass sie sich in einer magensäurebeständigen oralen Darreichungsform darbietet.

8. Zusammensetzung gemäss einem der Ansprüche 1 bis 7, dadurch gekennzeichnet, dass sie sich als biologisch abbaubare Mikrokugeln, Nanokugeln, Mikrokapseln oder Nanokapseln oder aber als lipidartige Vesikeln oder Liposomen darbietet.
